# EUROPEAN PATENT APPLICATION

(11) **EP 3 964 220 A1**
(43) Date of publication of application: **09.03.2022**
(21) Application number: 20305985.2
(22) Date of filing: 04.09.2020
(51) Int. Cl.: A61K 36/064, A61P 19/08, A61P 19/10, A61P 19/00

(54) **YEAST PRODUCT USEFUL FOR THE TREATMENT OF OSTEOPOROSIS**

(71) Applicant: Biocodex, 94250 Gentilly (FR)
(72) Inventor: VERLEYE, Marc, 60190 REMY (FR); LE GUERN, Marie-Emmanuelle, 60200 COMPIEGNE (FR)
(74) Representative: Vial, Lionel

(57) **Abstract**

The present invention relates to a yeast cell derived product for use for promoting osteoblastogenesis in an individual.

## Description

### Filed of the invention

The present invention relates to a pharmaceutical composition for promoting osteoblastogenesis.

### Background of the invention

Bone is a dynamic tissue that is constantly remodeled by osteoblasts, which are responsible of bone formation by producing matrix proteins and transporting mineral into the matrix, and osteoclasts, which are responsible of the resorption of the bone by breaking down the tissues.

Diseases of the bone are typically characterized by an imbalance of the remodeling process. By way of example, an acceleration of bone resorption not compensated by sufficient bone formation leads to an excessive loss of bone mass and will contribute to incidence of bone-related disorder.

Osteoporosis is the most common bone-related disorder in humans. It is characterized by low bone mass and micro-architectural deterioration of bone tissue due to an imbalance in the remodeling process with a consequent increase in bone fragility and susceptibility to fracture.

Fractures that occur spontaneously or following minor trauma, called fragility fractures, are very common in osteoporotic individuals. It is estimated that 50% of women and 20% of men over the age of 50 years will have an osteoporosis-related fracture in their remaining life. These fractures are responsible for lasting disability, impaired quality of life, social isolation, depression, and increased mortality.

Current therapies in the prevention of osteoporosis and fractures are designed to decrease bone resorption. They include estrogen, bisphosphonates, selective estrogen receptor modulators, and human monoclonal antibody against receptor activator of NF-κB ligand.

However, these treatments do not stimulate bone formation. Furthermore, some of these therapies have been reported to cause side effects. By way of example, bisphosphonates cause bone, joint, or muscle complaints, osteonecrosis of the jaw, and alendronate causing atypical fractures (Sozen et al. (2017) Eur J Rheumatol. 4(1): 46-56).

Accordingly, there is a need for alternative to current treatments of osteoporosis.

### Summary of the invention

The present invention arises from the unexpected finding by the inventors that, conditioned medium of *Saccharomyces boulardii* CNCM I-745 has a positive effect on the differentiation and mineralization process of osteoblasts in an in-vitro osteoblastogenesis protocol.

Thus, the present invention relates to a yeast cell-derived product for use for promoting osteoblastogenesis.

In an embodiment, the present invention relates to a yeast cell-derived product for use for preventing or treating a bone-related disorder.

In an embodiment of the invention, the yeast cell-derived product for use as defined above is in combination with at least one additional compound useful for the prevention or treatment of a bone-related disorder.

In an embodiment of the invention, the yeast cell-derived product for use as defined above is not in combination with butyric acid.

The present invention also relates to a pharmaceutical composition or medicament comprising a yeast cell-derived product as defined above as active substance, optionally in association with at least one pharmaceutically acceptable carrier or excipient, for use for promoting osteoblastogenesis.

In an embodiment of the invention, the above defined pharmaceutical composition or medicament further comprises at least one additional compound useful for the prevention or treatment of a bone-related disorder.

The present invention also relates to a pharmaceutical composition or medicament comprising:
- a yeast cell-derived product as defined above as active substance, and
- at least one additional compound useful for the prevention or treatment of a bone-related disorder,
optionally in association with at least one pharmaceutically acceptable carrier or excipient.

In an embodiment of the invention, the above defined pharmaceutical composition or medicament does not comprise butyric acid.

The present invention also relates to a method for promoting osteoblastogenesis in an individual, comprising administering to the individual an effective or prophylactic amount of a yeast cell-derived product as defined above.

In an embodiment of the method as defined above, the yeast cell-derived product is in association with at least one additional compound useful for the prevention or treatment of a bone-related disorder.

In an embodiment of the method as defined above, the yeast cell-derived product is not in association with butyric acid.

The present invention also relates to the use of a yeast cell-derived product for the preparation of a medicament intended to promote osteoblastogenesis.

In an embodiment of the use as defined above, the medicament comprises at least one additional compound useful for the prevention or treatment of a bone-related disorder.

In an embodiment of the use as defined above, the medicament does not comprise butyric acid.

The present invention also relates to products comprising:
- a yeast cell-derived product as defined above,
- at least one additional compound useful for the prevention or treatment of a bone-related disorder,
as a combined preparation for simultaneous, separated or sequential use for promoting osteoblastogenesis in an individual.

In an embodiment of the products as defined above, the products do not comprise butyric acid.

### Detailed description of the invention

On a preliminary basis, we recall that as intended herein, the term "comprising" has the meaning of "including" or "containing", which means that when an object "comprises" one or several elements, other elements than those mentioned may also be included in the object. In contrast, when an object is said to "consist of" one or several elements, the object is limited to the listed elements and cannot include other elements than those mentioned.

Besides, as one of skilled in the art will well understand, the expressions "substance or composition for use in the prevention or treatment of a disease" is equivalent to the expression "substance or composition for use in a method for the prevention or treatment of a disease".

### Osteoblostogenesis

As intended herein, the term "osteoblastogenesis" refers to the differentiation and formation of osteoblasts, in particular, to an increase of the presence and activity of osteoblasts.

Preferably, osteoblasts activity according to the invention encompasses bone formation and mineralization. Preferably, bone mineralization according to the invention preferably relates to calcium deposits in bone tissues.

Preferably, the present invention aims at promoting osteoblastogenesis, notably osteoblast activity, in particular to treat or prevent a bone-related disorder.

Preferably, the expression "bone-related disorder" refers to any disease, condition, disorder, syndrome, trauma of a bone or to a bone which would be benefited, treated, prevented or healed by osteoblastogenesis.

Preferably, a bone-related disorder according to the invention encompasses diseases linked or caused by the progressive loss of bone mass, diseases linked or cause by the progressive bone resorption and diseases linked or caused by an anormal bone mineralization, in particular a defect in mineralization.

The bone-related disorder according to the invention is preferably selected from the group consisting of osteoporosis, osteopenia, fracture and adult osteomalacia.

Osteoporosis is well known to one of skilled in the art and is notably defined in sections FB83.1 of the International Classification of Diseases 11th Revision of December 2018.

Ospetoporosis according to the invention notably includes:
- Premenopausal idiopathic osteoporosis
- Postmenopausal osteoporosis
- Osteoporosis of disuse
- Drug-induced osteoporosis
- Osteoporosis due to malabsorption
- Senile osteoporosis;
- Osteoporosis in multiple myelomatosis;
- Osteoporosis in endocrine disorders.

Osteopenia is well known to one of skilled in the art and is notably defined in sections FB83.00 of the International Classification of Diseases 11th Revision of December 2018.

Osteopenia according to the invention notably includes:
- Premenopausal idiopathic osteopenia
- Postmenopausal osteopenia
- Senile osteopenia
- Osteopenia of disuse
- Drug-induced osteopenia

Osteomalacia is well known to one of skilled in the art and is notably defined in sections FB83.2 of International Classification of Diseases 11th Revision of December 2018.

Osteomalacia according to the invention notably includes:
- Aluminium bone disease
- Adult osteomalacia due to malnutrition
- Drug-induced adult osteomalacia.

Fractures according to the invention encompasses fractures at any major skeletal site. Preferably, fractures according to the invention include osteoporosis-related fractures.

Preferably, fractures according to the invention includes:
- vertebral fractures;
- fractures of the femoral neck;
- fractures of the wrist;
- hip fractures;
- fractures of the shoulder;
- multiple fractures;
- Multiple vertebral thoracic fractures;
- Lumbar fractures.

### Yeast cell-derived product

A "yeast" according to the invention is a fungus, preferably unicellular. The yeast cells according to the invention are preferably of the genus *Saccharomyces*, more preferably of the species *Saccharomyces* cerevisiae and even more preferably of the species *Saccharomyces boulardii. Saccharomyces boulardii* is well known to one of skilled in the art and is notably described in Hennequin et al. (2001) J. Clin. Microbiol. 39:551-559.

Particularly preferably, the cells of *Saccharomyces boulardii* according to the invention are obtained from medicinal products of the brand Ultra-Levure^{®} or from deposits in the American Type Culture Collection (ATCC, USA) under reference 74012 or in the *Collection Nationale* de *Culture* et de *Microorganismes* (CNCM, France) under reference I-745.

Preferably, the yeast cells according to the invention are lyophilized, such as *Saccharomyces boulardii* yeast cells of the brand Ultra-Levure^{®}.

Advantageously, the viability and vitality of yeast cells obtained from lyophilizates are greater than can be obtained with other methods of preservation of yeast cells.

As understood here, "lyophilization" is a method of preservation in which the yeast cells are frozen and are then submitted to sublimation of the frozen water that they contain to give a lyophilizate in the form of dry yeast powder preferably containing less than 2% of water and more preferably less than 1% of water. Preferably, the lyophilized yeast cells are obtained from concentrates of yeast cells. Any type of method of lyophilization of yeast cells known by one of skilled in the art can be used. However, the yeast cells are preferably lyophilized according to the invention by means of the following method of lyophilization:
- cultivate the yeast cells in a liquid nutrient medium until the cells reach a stationary phase;
- concentrate the cultivated yeast cells and freeze the concentrate;
- lyophilize the concentrate.

As intended herein the expression "yeast cell-derived product" relates to any product which can be obtained from yeast cells in themselves orwhich contains yeast cells secretions. It is preferred that the yeast cell-derived product according to the invention is selected from the group consisting of yeast cells, a yeast cell culture, a yeast cell extract, a yeast cell conditioned medium and a yeast cell culture supernatant. More preferably, the yeast cell-derived product according to the invention is a yeast cell culture supernatant.

As understood herein, "yeast cells" or "yeast cell culture" include viable or dead yeast cells, intact or in the form of debris. Preferably, at least a proportion of the yeast cells according to the invention or comprised in the yeast cell culture according to the invention are viable, in particular viable and cultivable.

The viability of a yeast cell is defined as the capacity of a yeast cell to multiply. The viability of yeast cells can notably be determined by methylene blue coloration and microscopic observation. The number of viable cells and cultivable cells in a sample can be estimated by determining the number of Colony Forming Units (CFU) contained in the sample.

By way of example, the number of CFU of yeast cells in a liquid sample containing yeasts, such as a yeast cell culture, can be determined by spreading a specified volume of the sample on a solid medium, for example a gel medium, allowing the growth of yeasts, and incubating the solid medium for a period of time, for example 48 h, and at a temperature, for example 30°C, allowing the growth of yeast colonies. The number of colonies relative to the volume spread on the solid medium makes it possible to determine the number of CFU contained in the sample. A detailed protocol for determination of CFU according to the invention is notably described in Toothaker and Elmer (1984) Antimicrobial Agents and Chemotherapy 26:552-556 in the paragraph "Assay *for S. boulardii*". Moreover, when the yeast sample is in the form of a solid, for example a lyophilized powder, it is preferred to determine the number of CFU contained in the sample after solvating a specified mass of the sample in an aqueous solution, notably distilled water or a 0.9% NaCl solution at pH 7.

Preferably, yeast cells or a yeast cell culture according to the invention comprise from 10⁸ UFC/g to 10¹² UFC/g, more preferably, from 2.10⁹ UFC/g to 2.10¹¹ UFC/g.

Numerous methods for preparing yeast cells, a yeast cell culture, a yeast cell extract, a yeast cell conditioned medium and a yeast cell culture supernatant are well known in the art and are notably described in "Yeast Protocols" (1996) Methods in Cell and Molecular Biology, Ed. Ivor H. Evans, Humana Press.

The yeast cell culture according to the invention can be obtained by any standard method well known to one of skilled in the art for culturing yeast cells and for instance as described in the Example section. By way of example the yeast cell culture according to the invention can be obtained by inoculating a complete liquid culture medium such as yeast extract peptone dextrose (YEPD or YPD) and incubating the medium at 30°C - 37°C under agitation and aerobic conditions during at least 24 hours, 36 hours, or 48 hours and preferably less than 96 hours.

Yeast cells according to the invention can be obtained from a yeast cell culture medium according to the invention through sedimentation or centrifugation of the yeast cells.

Yeast cell extracts according to the invention can be obtained by any yeast cell fragmentation method known in the art applied to the yeast cell culture or the yeast cells according to the invention, such as autolysis, hydrolysis or autoclaving. In particular, the yeast cells extract according to the invention is selected from the group consisting of a membrane extract, a cytoplasmic extract or a nuclear extract.

Preferably, the yeast cell-conditioned medium according to the invention relates to any medium, such as a liquid cell culture medium, which has been contacted by yeast cells. Preferably, the medium has been contacted by yeast cells for a time sufficient for the yeast cells to have secreted in the medium. By way of example, the medium has been contacted by yeast cells during 1 to 9 days, preferably 1, 2, 3, 4, 5, 6, 7, 8 or 9 days. The conditioned medium according to the invention preferably contains molecules secreted by the yeast cells such as proteins. Preferably, the conditioned medium according to the invention contains no cellular debris.

The yeast cell culture supernatant can be obtained by any method well known to one of skilled in the art. The yeast cell culture supernatant according to the invention may notably be obtained by centrifugation of the yeast cell culture according to the invention and taking the supernatant part of the centrifugated culture. The yeast cell culture supernatant according to the invention may also be obtained by filtrating the yeast cell culture according to the invention through a filter retaining yeast cells and recuperating the filtrate.

The yeast cell culture, the yeast cell extract, the yeast cell-conditioned medium and the yeast cell culture supernatant according to the invention may have been subjected to at least one treatment or processing step such as centrifugation, filtration, purification, chromatography, concentration, decantation, heating, pasteurisation, autoclaving, drying, freeze-drying or distillation, in particular filtration, more particularly sterilizing filtration for example with 0.2 µm filters.

In another embodiment, the yeast cell-derived product according to the invention contains living cells that allow a long-lasting action.

In another embodiment, the yeast cell-derived product according to the invention does not contain living cells.

Preferably, the yeast cell-derived product for use according to the invention comprises:
- taking yeast cells,
- culturing the yeast cells,
- obtaining a yeast cell-derived product, and
- administering the yeast cell-derived product to an individual.

Preferably, the yeast cell-derived product according to the invention is for an administration or is administered at a dosage of from 0.5x10⁸ to 100x10¹⁰ CFU/kg/d or at a dose of from 0.00125 g/kg/d to 25 g/kg/d.

Preferably, the yeast cell-derived product according to the invention is administered at a dose of from 0.5 g/kg to 10 g/kg, more preferably at a dose of from 1 to 6 g/kg and even more preferably at a dose of about 3 g/kg, in particular once, twice, three times, four times or five times per day.

As one of skill in the art will understand, the quantity of yeast cell-derived product to administer by mass unit (kg) relates to the mass of the individual to whom the yeast cell-derived product is intended to be administered. The quantity of yeast cell-derived product expressed in UFC or in g, refers to the quantity of yeast cells administered where the yeast cell-derived product is yeast cells or a yeast cell culture, or to the quantity of yeast cells from which the yeast cell extract, the yeast cell culture supernatant or the yeast cell conditioned medium is prepared. When the quantity of yeast cell-derived product is expressed in mass unit (g), the yeast cell product is preferably under dried or lyophilized (freeze-dried) form, more preferably under lyophilized form. When the yeast cell-derived product is administered in liquid form, it is preferably administered in a volume of from 1 µL to 10 mL, more preferably 100 µL to 2 mL, optionally after prior concentration or dilution.

### Additional compound

The additional compound useful for the prevention or treatment of bone-related disorder can be of any type well known to one of skill in the art.

Preferably, the additional compound according to the invention is selected from the group consisting of selective estrogen receptor modulator, anabolic drug, bisphosphonates, hormones, vitamins, calcium supplements, and monoclonal antibody which block osteoclasts.

Preferably, the selective estrogen receptor modulator is raloxifene.

Preferably, the anabolic drug is Teriparatide.

Preferably, the bisphosphonates are selected from the group consisting of alendronate, risedronate, etidronate, ibandronic acid, zoledronic acid, bonviva, and aclasta.

Preferably, hormones are selected form the group consisting of calcitonin, tamoxifen, and synthetic parathormone.

Preferably, vitamins are vitamin D.

Preferably, the monoclonal antibody which block osteoclasts is denosumab.

Preferably, yeast cell-derived product according to the invention are administered with analgesics to relive pain.

### Administration

As intended herein, "combined" or "in combination" means that the yeast cell-derived product, as defined above, is administered at the same time than another compound or product, either together, i.e. at the same administration site, or separately, or at different times, provided that the time period during which the yeast cell-derived product, as defined above, exerts its effects on the individual and the time period during which the additional agent or product exerts its pharmacological effects on the individual, at least partially intersect.

Preferably, the yeast cell-derived product according to the invention is administered in a prophylactically or therapeutically effective amount for promoting osteoblasogenesis, in particular osteoblast activity, more particularly bone formation.

Preferably, also the yeast cell-derived product according to the invention is administered in a prophylactically or therapeutically effective amount for preventing or treating a bone-related disorder.

The administration of the yeast cell-derived product or the pharmaceutical composition or medicament comprising the yeast cell-derived product according to the invention can proceed by any method known in the art.

Preferably, the yeast cell-derived product or the pharmaceutical composition or medicament comprising the yeast cell-derived product according to the invention is in a form suitable for being administered or is administered subcutaneously, intravenously, intramuscularly, intra-dermally, topically or by the oral route, the parenteral route, the intradermal route, the intravenous route, the arterial route, the intramuscular route, the nasal route, the rectal or the subcutaneous route.

Preferably, the yeast cell-derived product or the pharmaceutical composition or medicament comprising the yeast cell-derived product according to the invention is in the form of a powder, a sachet, a tablet, a capsule, a patch, or a liquid or gel solution or an aerosol.

### Pharmaceutical composition

As intended herein "pharmaceutically acceptable carrier or excipient "refers to any material suitable with a pharmaceutical composition. Preferably, the pharmaceutically acceptable carrier or excipient according to the invention is suitable for a yeast cell-derived product according to the invention in the form of a solid, a liquid or an aerosol.

Preferably, the pharmaceutically acceptable carrier or excipient according to the invention is suitable for an administration by the oral route, the parenteral route, the intradermal route, the intravenous route, the arterial route, the intramuscular route, the nasal route, the rectal or the subcutaneous route.

Preferably, the pharmaceutically acceptable carrier or excipient according to the invention, includes but is not limited to any of the standard carrier or excipient known to one of skilled in the art such as water, glycerin, alcohol, oil emulsion, water emulsion, buffered saline solution, preservative, stabilizer and wetting agents.

### Individual

As intended herein the individual according to the invention is preferably a mammal, more preferably a human. Preferably, also the individual according to the invention is a female.

Preferably, the individual according to the invention is more than 45, 55, 65 or 80 years old.

Preferably, the individual according to the invention presents a loss of bone density and/or a bone resorption.

Preferably also the individual according to the invention in an individual with a bone-related disorder.

Preferably also, the individual according to the invention is an individual with osteoporosis, osteopenia, a fracture or osteomalacia.

Preferably, the individual according to the invention is an individual with a fracture selected form the group consisting of an osteoporosis-related fracture, a vertebral fracture, a fracture of the femoral neck, a fracture of the wrist, a hip fracture, a fracture of the shoulder, a multiple fracture, a multiple vertebral thoracic fractures, and a lumbar fractures.

Preferably, the induvial according to the invention does not have a bone-related disorder.

The invention will be further explained with the following Figures and non-limiting Examples.

### Description of the figures

Figure 1

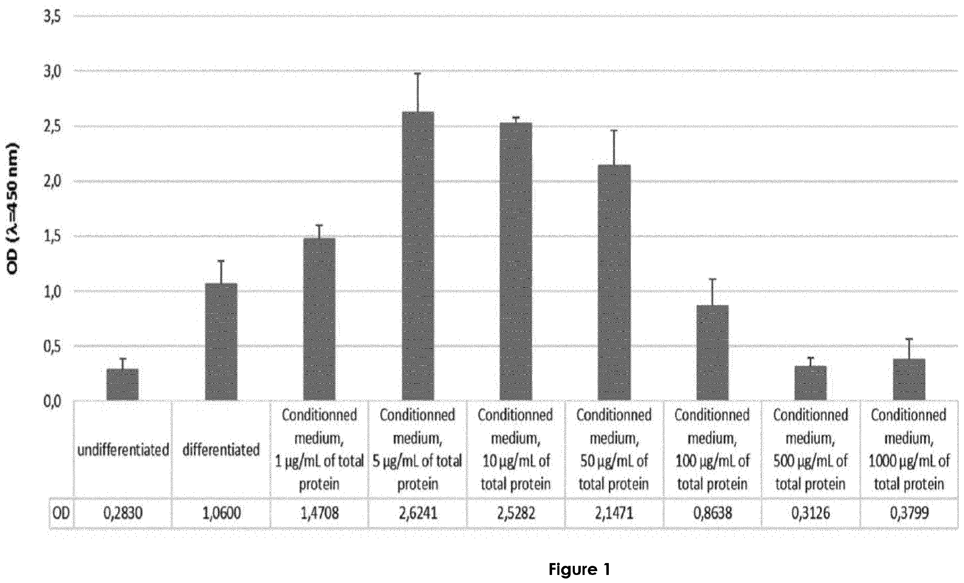

   Figure 1 shows the measurement of the amount of staining by optical density for culture of human MSCs undifferentiated, differentiated, differentiated and treated with the conditioned medium of *Saccharomyces boulardii* CNCM I-745 at 1 µg/mL, 5 µg/mL, 10 µg/mL, 50 µg/mL, 100 µg/mL, 500 µg/mL and 1000 µg/mL. Data are expressed as mean optical density ± SD from n = 2 replicates.
Figure 2

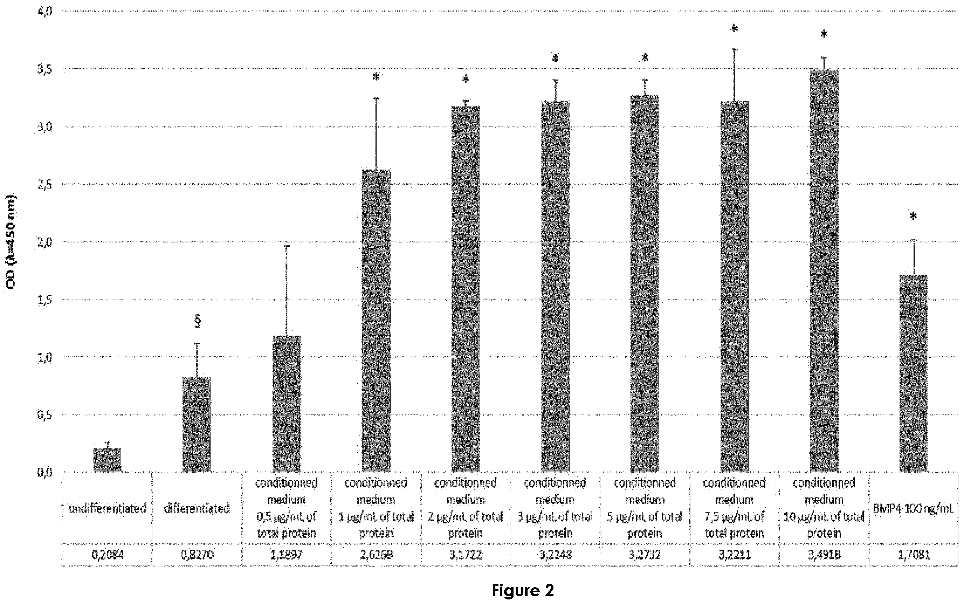

   Figure 2 shows the measurement of optical density of staining by optical density for culture of human MSCs undifferentiated, differentiated, and differentiated and treated with the conditioned medium of *Saccharomyces boulardii* CNCM I-745 batch A at 0,5 µg/mL, 1 µg/mL, 2 µg/mL, 3 µg/mL, 5 µg/mL, 7.5 µg/mL, and 10 µg/mL, or BMP-4. Data are expressed as mean optical density ± SD from n = 4 replicates.
   The statistical significance of the difference between undifferentiated cells and differentiated cells and of the difference between differentiated cells and differentiated cells treated with the conditioned medium or BMP-4 was determined using the Mann Whitney test. § p< 0.05 vs undifferentiated; *p<0.05 vs differentiated.
Figure 2

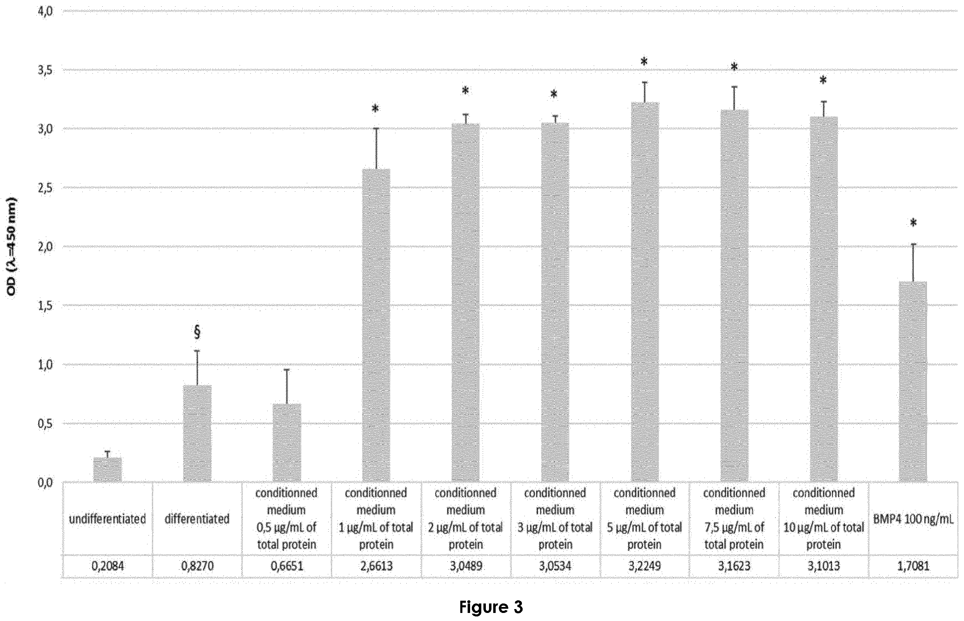

   Figure 3 discloses discloses the measurement of optical density of staining by optical density for culture of human MSCs undifferentiated, differentiated, and differentiated cells treated with the conditioned medium of *Saccharomyces boulardii* CNCM I-745 batch B at 0,5 µg/mL, 1 µg/mL, 2 µg/mL, 3 µg/mL, 5 µg/mL, 7.5 µg/mL, and 10 µg/mL, or BMP-4. Data are expressed as mean optical density ± SD from n = 4 replicates.
   The statistical significance of the difference between undifferentiated cells and differentiated cells and of the difference between differentiated cells and differentiated cells treated with the conditioned medium or BMP-4 was determined using the Mann Whitney test. § p< 0.05 vs undifferentiated; *p<0.05 vs differentiated.

### Example 1

The inventors have evaluated the efficacy of a conditioned medium of *Saccharomyces boulardii* in an *in vitro* osteoblastogenesis protocol.

### 1. Material and methods

### 1.1. Conditioned media of Saccharomyces boulardii CNCM 1-745

- Protein concentration: 2,5 mg/mL (batch 1), 2,8 mg/mL (batch 2) and 2,5 mg/mL (batch 3)
- Storage: + 4 °C

Each new conditioned medium is diluted in DMEM in order to adjust the protein concentration at 2 mg/mL. Stock solutions are stored at + 4°C for a maximum of one week.

### 1.2. In vitro osteoblastogenesis protocol from human mesenchymal stem cells (hMSCs)

Human MSCs supplied by RoosterBio (Ref.: KT-014, Batch: 00137, Donor: Male 22 years) are differentiated into osteoblasts in the presence of vitamin D3 (10⁻⁸ M), dexamethasone (10⁻⁸ M), ascorbic acid (50 µg/mL) and β-glycerophosphate (10 mM) (culture in differentiation medium: DMEM medium supplemented with 5% FBS, vitamin D3, dexamethasone, ascorbic acid and β-glycerophosphate). The differentiation time depends on the donor; it is on average 21 days after start of differentiation. Here, osteoblastogenesis is stopped at day 17. Cells in DMEM 5% FBS serve as a negative control (basal medium).

### 1.4. Protocol

### 1.4.1. Seeding, differentiation and treatment of MSCs

Human MSCs are thawed. Cells are seeded and cultured in flask in the medium for cell proliferation (RoosterBio, KT-001). 4 days after thawing, human MSCs are detached with trypsin-EDTA and counted. The cells are seeded at 2.105 cells per well and cultured in monolayer in 24-well plates in DMEM medium supplemented with 5% FBS (basal medium) for four days (the day of seeding is designated day -4).

The osteoblastogenesis is carried out in 24-well culture plates. All treatments and controls are carried out in duplicate. After four days of culture in basal medium, treatments are applied over the differentiation process (the first day of treatment is designated day 0).

The following 9 conditions are carried out:
- Cells + basal medium → negative control
- Cells + differentiation medium → positive control
- Cells + differentiation medium + conditioned medium (1000 µg/mL of total protein)
- Cells + differentiation medium + conditioned medium (500 µg/mL of total protein)
- Cells + differentiation medium + conditioned medium (100 µg/mL of total protein)
- Cells + differentiation medium + conditioned medium (50 µg/mL of total protein)
- Cells + differentiation medium + conditioned medium (10 µg/mL of total protein)
- Cells + differentiation medium + conditioned medium (5 µg/mL of total protein)
- Cells + differentiation medium + conditioned medium (1 µg/mL of total protein).

Differentiation medium is basal medium supplemented with vitamin D3 (10⁻⁸ M) and dexamethasone (10⁻⁸ M) for four days and then with vitamin D3 (10⁻⁸ M), dexamethasone (10⁻⁸ M), ascorbic acid (50 µg/mL) and β-glycerophosphate (10 mM) until the end of the differentiation process.

Medium completed with appropriate conditions of treatment are changed every 3-4 days. At day 14, a well-developed mineralization is observed in the culture of untreated differentiated cells. The test is stopped. Presence of calcium deposits is highlighted by a red alizarin staining. The amount of mineral matrix is quantified by spectrophotometry after dissolution of the stained matrix.

### 1.4.2. Preparation and differentiation medium 2X

At day 0, the differentiation medium 2X is prepared by diluting in DMEM 10% FBS at 1:500 a 10⁻⁵M solution of dexamethasone (final concentration 2.10⁻⁸M) and a 10⁻⁵M solution of vitamin D3 (final concentration 2.10⁻⁸M).

At day 4 and then every 3-4 days, the differentiation medium 2X is prepared by diluting in DMEM 10% FBS:
- at 1:500 a 10⁻⁵M solution of dexamethasone (final concentration 2.10⁻⁸ M) and a 10⁻⁵ M solution of vitamin D3 (final concentration 2.10⁻⁸M).
- at 1:50 a 5 mg/mL solution of ascorbic acid and a 1 M solution of β-glycerophosphate respectively (final concentration, ascorbic acid 100 µg/mL, β-glycerophosphate 20 mM).

### 1.4.3. Preparation and treatment with conditioned media

At day 0 and then every 3-4 days, the 2 mg/mL stock solution is diluted in DMEM medium in order to prepare intermediate solutions at 1000 µg/mL, 200 µg/mL, 100 µg/mL, 20 µg/mL, 10 µg/mL and 2 µg/mL. Then, stock solution and intermediate solutions are diluted at 1:2 in differentiation medium 2X* in order to treat cells with differentiation medium + 1000 µg/mL, 500 µg/mL, 100 µg/mL, 50 µg/mL, 10 µg/mL, 5 µg/mL and 1 µg/mL of total protein of the conditioned medium.

| **Starting concentration** | **Volume** | **DMEM** | **Dilution** | **Final concentration (2X)** | **Final volume** |
|---|---|---|---|---|---|
| **2000 µg/mL [stock solution]** | 500 µL | 500 µL L | 1/2 | 1000 µg/mL | 1000 µL |
| **1000 µg/mL** | 200 µ L | 800 µ L | 1/5 | 200 µg/mL | |
| **200 µg/mL** | 500 µ L | 500 µ L | 1/2 | 100 µg/mL | |
| **100 µg/mL** | 200 µ L | 800 µ L | 1/5 | 20 µg/mL | |
| **20 µg/mL** | 500 µ L | 500 µ L | 1/2 | 10 µg/mL | |
| **10 µg/mL** | 200 µ L | 800 µ L | 1/5 | 2 µg/mL | |

| | | | | | |
|---|---|---|---|---|---|
| (^{*}) Differentiation medium 2X: DMEM + 10% FBS + 2.10⁻⁸ M dexamethasone + 2.10⁻⁸ M vitamin D3 + 100 µg/mL ascorbic acid + 20 mM β-glycerophosphate. | | | | | |

Treatments are prepared fresh prior to use on the day of treatment and not stored for testing at a later date.

### 1.5. Data recording and processing

The Multiskan spectrophotometer plate reader is used for optical density (OD) measurement. 100% of data are verified for the transfer of the values generated by the multiskan software (optical density 450 nm) to the corresponding table for graphs. 10% of all additional raw data (e.g., culture data, staining, etc.) are verified.

### 2. Results

Over the course of the culture, no sign of toxicity is noticed using microscopic observation. At day 14, a well-developed mineralization is observed in the positive control, the experiment is stopped and the red alizarin staining is performed.

As shown in **Figure 1****,** the amount of red alizarin staining for the differentiated cells is significantly higher than the amount of staining for the undifferentiated cells. An increase of the amount of red alizarin staining is noticed in the wells treated with conditioned medium at 1 µg/mL, 5 µg/mL, 10 µg/mL and 50 µg/mL compared to the positive control. The increase is dose-dependent.

As such, conditioned medium of yeast cells have a positive effect on the differentiation and mineralization of osteoblasts in an *in vitro* osteoblastogenesis protocol.

### Example 2

The inventors have evaluated the effect of two distinct batches of conditioned medium of *Saccharomyces boulardii* CNCM I-745 in an *in vitro* osteoblastogenesis protocol.

### 1. Material and methods

### 1.1. Conditioned media of Saccharomyces boulardii CNCM 1-745

- Protein concentration batch A: 2,41 mg/mL (batch A1), 2,53 mg/mL (batch A2) and 2,36 mg/mL (batch A3);
- Protein concentration batch B: 2,41 mg/mL (batch B1), 2,43 mg/mL (batch B2) and 2,26 mg/mL (batch B3);
- Storage: + 4 °C.

Each new conditioned medium is diluted in DMEM in order to adjust the protein concentration at 2 mg/mL. Stock solutions are stored at + 4°C for a maximum of one week.

### 1.2. Recombinant human Bone Morphogenetic Protein-4 (BMP-4)

BMP-4 (R&D systems, ref: 314-BP, batch: BEM11817121, supplied as a lyophilized powder) is stored at -20°C.

For the preparation of a stock solution of 100 µg/mL, BMP-4 is dissolved in 4 mM HCI containing 0,1 % BSA. The stock solution is stored in aliquots at -20°C. Repeated freeze-thaw cycles are avoided.

### 1.3. In vitro osteoblastogenesis protocol from human mesenchymal stem cells (hMSCs)

Human MSCs (RoosterBio, Ref. MSC-003, Batch: 00178, donor: Male 22 years) are differentiated into osteoblasts in the presence of vitamin D3 (10⁻⁸ M), dexamethasone (10⁻⁸ M), ascorbic acid (50 µg/mL) and β-glycerophosphate (10 mM) (culture in differentiation medium: DMEM medium supplemented with 5% FBS, vitamin D3, dexamethasone, ascorbic acid and β-glycerophosphate). The differentiation time depends on the donor; it is on average 21 days after start of differentiation. Here, osteoblastogenesis is stopped at day 14. Cells in DMEM 5% FBS serve as a negative control (basal medium).

### 1.4. Protocol

Human MSCs are thawed. Cells are seeded and cultured in flask in the medium for cell proliferation (RoosterBio, KT-001). 4 days after thawing, human MSCs are detached with trypsin-EDTA and counted. The cells are seeded at 2.105 cells per well and cultured in monolayer in 24-well plates in DMEM medium supplemented with 5% FBS (basal medium) for four days (the day of seeding is designated day -4).

The osteoblastogenesis is carried out in 24-well culture plates. All treatments and controls are carried out in duplicate. After four days of culture in basal medium, treatments (BMP-4 or conditioned medium/*Saccharomyces boulardii* CNCM I-745) are applied over the differentiation process (the first day of treatment is designated day 0).

The following 17 conditions are carried out:
- Cells + basal medium → negative control
- Cells + differentiation medium → positive control
- Cells + differentiation medium + BMP4 → BMP control
- Cells + differentiation medium + conditioned medium / *Saccharomyces boulardii* CNCM I-745 batch A (10 µg/mL of total protein)
- Cells + differentiation medium + conditioned medium / *Saccharomyces boulardii* CNCM I-745 batch A (7,5 µg/mL of total protein)
- Cells + differentiation medium + conditioned medium / *Saccharomyces boulardii* CNCM I-745 batch A (5 µg/mL of total protein)
- Cells + differentiation medium + conditioned medium / *Saccharomyces boulardii* CNCM I-745 batch A (3 µg/mL of total protein)
- Cells + differentiation medium + conditioned medium / *Saccharomyces boulardii* CNCM I-745 batch A (2 µg/mL of total protein)
- Cells + differentiation medium + conditioned medium / *Saccharomyces boulardii* CNCM I-745 batch A (1 µg/mL of total protein)
- Cells + differentiation medium + conditioned medium / *Saccharomyces boulardii* CNCM I-745 batch A (0,5 µg/mL of total protein)
- Cells + differentiation medium + conditioned medium / *Saccharomyces boulardii* CNCM I-745 batch B (10 µg/mL of total protein)
- Cells + differentiation medium + conditioned medium / *Saccharomyces boulardii* CNCM I-745 batch B (7,5 µg/mL of total protein)
- Cells + differentiation medium + conditioned medium / *Saccharomyces boulardii* CNCM I-745 batch B (5 µg/mL of total protein)
- Cells + differentiation medium + conditioned medium / *Saccharomyces boulardii* CNCM I-745 batch B (3 µg/mL of total protein)
- Cells + differentiation medium + conditioned medium / *Saccharomyces boulardii* CNCM I-745 batch B (2 µg/mL of total protein)
- Cells + differentiation medium + conditioned medium / *Saccharomyces boulardii* CNCM I-745 batch B (1 µg/mL of total protein)
- Cells + differentiation medium + conditioned medium / *Saccharomyces boulardii* CNCM I-745 batch B (0,5 µg/mL of total protein)

At day 0, the differentiation medium 2X is prepared by diluting in DMEM 10% FBS at 1:500 a 10⁻⁵M solution of dexamethasone (final concentration 2.10⁻⁸ M) and a 10⁻⁵ M solution of vitamin D3 (final concentration 2.10⁻⁸ M).

At day 4 and then every 3-4 days, the differentiation medium 2X is prepared by diluting in DMEM 10% FBS:
- at 1:500 a 10⁻⁵ M solution of dexamethasone (final concentration 2.10⁻⁸ M) and a 10⁻⁵M solution of vitamin D3 (final concentration 2.10⁻⁸ M).
- at 1:50 a 5 mg/mL solution of ascorbic acid and a 1 M solution of β-glycerophosphate respectively (final concentration, ascorbic acid 100 µg/mL, β-glycerophosphate 20 mM).

The protein concentration of the batches is adjusted at 2 mg/mL (stock solution). Every batch is used for two medium renewal. At day 0 and then every 3-4 days, the 2 mg/mL stock solution is diluted in DMEM medium in order to prepare intermediate solutions at 20 µg/mL, 15 µg/mL, 10 µg/mL, 6 µg/mL, 4 µg/mL, 2 µg/mL and 1 µg/mL. Then, intermediate solutions are diluted at 1:2 in differentiation medium 2X(a) in order to treat cells with differentiation medium + 10 µg/mL, 7.5 µg/mL, 5 µg/mL, 3 µg/mL, 2 µg/mL, 1 µg/mL and 0.5 µg/mL of total protein of the conditioned medium.

| **Starting concentration** | **Volume** | **DMEM** | **Dilution** | **Final concentration (2X)** | **Final volume** |
|---|---|---|---|---|---|
| **2000 µg/mL [stock solution]** | 43 µ L | 4257 µ L | 1/100 | 20 µg/mL | 4300 µ L |
| **20 µg/mL** | 1125 µL | 375 µL | 1/1,3 | 15 µg/mL | 1500 µL |
| **20 µg/mL** | 750 µ L | 750 µ L | 1/2 | 10 µg/mL | |
| **20 µg/mL** | 450 µ L | 1050 µL | 1/3,3 | 6 µg/mL | |
| **20 µg/mL** | 300 µ L | 1200 µL | 1/5 | 4 µg/mL | |
| **20 µg/mL** | 150 µL | 1350 µL | 1/10 | 2 µg/mL | |
| **20 µg/mL** | 75 µL | 1425 µL | 1/20 | 1 µg/mL | |

| | | | | | |
|---|---|---|---|---|---|
| (a) Differentiation medium 2X: DMEM + 10% FBS + 2.10⁻⁸ M dexamethasone + 2.10⁻⁸ M vitamin D3 + 100 µg/mL ascorbic acid + 20 mM β-glycerophosphate. | | | | | |

Treatments are prepared fresh prior to use on the day of treatment and not stored for testing at a later date.

### 1.5. Data recording and processing

The MULTISKAN^{™} spectrophotometer plate reader (Thermo Fisher Scientific) is used for optical density measurement. 100% of data are verified for the transfer of the values generated by the MUMTISKAN^{™} software (optical density at 450 nm) to the corresponding table for graphs. 10% of all additional raw data (e.g., culture data, staining, etc.) are verified.

### 2. Results

In order to determine the effect of conditioned medium of *Saccharomyces boulardii* CNCM I-745 on *in vitro* human osteoblastogenesis, human MSCs are differentiated with or without the conditioned medium and the amount of mineralized matrix is quantified. At day 14, a well-developed mineralization is observed in the positive control, the experiment is stopped and the red alizarin staining is performed.
As shown in **Figure 1** **and** **Figure 2****,** the amount of red alizarin staining for the differentiated cells is significantly higher than the amount of staining for the undifferentiated cells. A significant increase of the amount of red alizarin staining is also observed in the wells treated with BMP-4 at 100 ng/mL, chosen as positive reference, compared to the positive control. Whatever the batch of yeast, an increase of the amount of red alizarin staining is noticed in the wells treated with conditioned medium for concentrations between 1 µg/mL and 10 µg/mL compared to the positive control. The increase is dose-dependent.

As such, conditioned medium of yeast cells have a positive effect on the differentiation/mineralization process of osteoblast in an osteoblastogenesis protocol, for concentrations between 1 µg/mL and 10 µg/mL. Indeed, a higher amount of calcium deposits is observed in the wells treated with conditioned medium at these concentrations compared to untreated differentiated cells.

## Claims

1. A yeast cell derived product for use for promoting osteoblastogenesis in an individual.

2. The yeast cell-derived product for use according to claim 1, for increasing bone formation and/or calcium deposit in bone tissues.

3. The yeast cell-derived product for use according to claim 1 or 2, for the prevention or treatment of a bone-related disorder.

4. The yeast cell-derived product for use according to claim 4, wherein the bone-related disorder is selected from the group consisting of osteoporosis, osteopenia, fracture, and osteomalacia.

5. The yeast cell-derived product for use according to any one of claims 1 to 4, wherein the yeast cell-derived product is selected from the group consisting of yeast cells, a yeast cell culture, a yeast cell extract, a yeast cell conditioned medium and a yeast cell culture supernatant.

6. The yeast cell-derived product for use according to any one of claims 1 to 5, wherein the yeast cell-derived product is derived from yeast cells of the genus *Saccharomyces*.

7. The yeast cell-derived product for use according to any one of claims 1 to 6, wherein the yeast cell-derived product is derived from yeast cells of the species *Saccharomyces boulardii.*

8. The yeast cell-derived product for use according to any one of claims 1 to 7, wherein the yeast cell-derived product is prepared from lyophilized yeast cells.

9. The yeast cell-derived product for use according to any one of claims 1 to 8, wherein the yeast cell-derived product is in a form suitable for an administration or is administered by the, the oral route, the rectal route, the topical route, the nasal route, the parenteral route, the intradermal route, the intravenous route, the arterial route, the intramuscular route or the subcutaneous route.

10. The yeast cell-derived product for use according to any one of claims 1 to 9, wherein the yeast cell-derived product is in combination with at least one additional compound useful for the prevention or treatment of a bone-related disorder.

11. The yeast cell-derived product for use according to claim 10, wherein the at least one additional compound useful for the prevention or treatment of a bone-related disorder is selected from the group consisting of specific oestradiol receptor modulator, anabolic drug, bisphosphonates, hormones, vitamins, calcium supplements, and monoclonal antibody which block osteoclasts.

12. A pharmaceutical composition comprising a yeast cell-derived product as defined in any of claims 1 to 8 as active substance, and optionally at least one pharmaceutically acceptable carrier or excipient, for use for promoting osteoblastogenesis according to any one of claims 1 to 4.

13. A pharmaceutical composition comprising:
- a yeast cell-derived product as defined in any of claims 1 to 8 as active substance, and
- at least one additional compound useful for the prevention or treatment of a bone-related disorder as defined in claim 10 or 11, and
optionally at least one pharmaceutically acceptable carrier or excipient.

14. The pharmaceutical composition according to claim 13, for use for promoting osteoblastogenesis according to any one of claims 1 to 4.

15. Products comprising:
- a yeast cell-derived product as defined in any one of claims 1 to 8,
- at least one additional compound useful for the prevention or treatment of a bone-related disorder as defined in claim 10 or 11,
as a combined preparation for simultaneous, separated or sequential use for promoting osteoblasogenesis according to any one of claims 1 to 4 in an individual.
